# EUROPEAN PATENT APPLICATION

(11) **EP 4 209 579 A1**
(43) Date of publication of application: **12.07.2023**
(21) Application number: 21864360.9
(22) Date of filing: 01.09.2021
(51) Int. Cl.: C12N 1/20, A23L 2/38, A23L 2/52, A23L 33/135, C12N 15/11

(54) **BIFIDOBACTERIUM INHIBITING EXPRESSION OF MUSCLE ATROPHY GENE**

(30) Priority: 02.09.2020 JP 2020147207
(71) Applicant: NISSIN FOODS HOLDINGS CO., LTD., Yodogawa-ku Osaka-shi, Osaka 532-8524 (JP)
(72) Inventor: IGI, Akemi, Osaka-shi, Osaka 532-8524 (JP); SUNADA, Yosuke, Osaka-shi, Osaka 532-8524 (JP); TANII, Yusuke, Osaka-shi, Osaka 532-8524 (JP); UEHARA, Kazuya, Osaka-shi, Osaka 532-8524 (JP); MORI, Ayaka, Osaka-shi, Osaka 532-8524 (JP)
(74) Representative: Mewburn Ellis LLP
(86) International application number: PCT/JP2021/032091
(87) International publication number: WO 2022/050303

(57) **Abstract**

The present invention provides a bifidobacterium derived from infant intestines which the bifidobacterium supresses expression of a muscle atrophy gene and can be suitably used in a food and beverage, and a foods and beverage containing the bifidobacterium. *Bifidobacterium breve* strain N106 (NITE BP-03231) is provided that suppresses the expression of the muscle atrophy gene. The muscle atrophy gene is a MuRF1 gene. Also provided is a food and beverage containing the bifidobacterium. Even persons with exercise difficulties can thereby prevent muscle atrophy and improve the quality of life through diet.

## Description

### TECHNICAL FIELD

The present invention relates to a strain of *Bifidobacterium* derived from infant intestines which strain suppresses the expression of a muscle atrophy gene, and food and beverage products containing the strain. In particular, the present invention relates to *Bifidobacterium breve.*

### BACKGROUND ART

The World Health Organization (WHO) defines a society of aged 65 years or older from 7% to less than 14% as an aging society, from 14% to less than 21% as an aged society, and 21% or more as a super-aged society among the total population. According to the data released by the Ministry of Internal Affairs and Communications (Japan) in 2019, the number of elderly people aged 65 and over in Japan is estimated at 35.88 million, accounting for 28.4% of the total population. Current Japan thus falls under the category of a super-aged society. The reason for the aging of the population is assumed to be an increase in average life expectancy due to medical advances and other factors.

Even if the average life expectancy increases, the body senesces with advancing age. One example aging process is the loss of muscle strength (muscle mass). Muscle is formed through the balance between synthesis and degradation. PGC-1α and MuRF1 genes are known to be involved in muscle synthesis and muscle degradation, respectively. MuRF1 has been shown to induce degradation of the "myosin heavy chain" that constitutes skeletal muscle tissue. If muscle degradation exceeds muscle synthesis, muscle degradation will progress and muscle mass will decrease. As muscle degradation further progresses, muscles atrophy (myatrophy).

A certain degree of loss of muscle mass is diagnosed as sarcopenia. In particular, if the mass of skeletal muscles, which are distributed along the skeleton and support physical activity, decreases, people cannot move their bodies as they should, increasing the risk of fractures due to falls, hospitalization, and being bedridden. People accordingly need to prevent the loss of muscle mass in order to lead healthy and cultured lives.

Habitual exercise is recommended to prevent loss of muscle mass. However, it is difficult to continue sustained exercise for those whose muscles have atrophied due to aging, those who are bedridden or unable to strain their motor organs (bones and joints) and circulatory organs, not limited to the elderly, and those who are busy every day. As a result, it is difficult to sufficiently restore the muscles of a person who cannot exercise even if he or she wishes to do so. In addition, when a person has been immobilized in a cast due to an injury or other reasons, the muscles contract and atrophy of the muscles is accelerated.

### CITATION LIST

### PATENT LITERATURE

[PTL 1] JP-2005-154387-A

### SUMMARY OF INVENTION

### TECHNICAL PROBLEM

The present invention has been made in view of the above problems. An object of the present invention is to provide a method of preventing muscle atrophy by means other than exercise.

### SOLUTION TO PROBLEM

The inventors have focused on MuRF1, which is a known muscle atrophy gene, and have examined suppression of MuRF1 expression by diet. As a result, the inventors have discovered existence of a bifidobacterium that supressed the expression of MuRF1 among bifidobacteria isolated from infant feces, and have completed the present invention.

To solve the above problem, the present invention is characterized by a bifidobacterium belonging to the *Bifidobacterium* genus *breve* species that suppresses the expression of the muscle atrophy gene. The muscle atrophy gene in the present invention is a concept that includes a gene sequence encoding a protein involved in muscle atrophy, whether directly or indirectly. The present invention is also characterized in that the muscle atrophy gene is MuRF1.

To solve the above problem, the present invention is also characterized in that the bifidobacterium is *Bifidobacterium breve* strain N106 (NITE BP-03231).

To solve the above problem, the present invention is further characterized by a food and beverage containing the bifidobacterium.

### ADVANTAGEOUS EFFECTS OF INVENTION

The bifidobacterium of this invention suppresses the expression of a muscle atrophy gene. This allows even those who have difficulty exercising to prevent muscle atrophy and to improve their quality of life through diet. In addition, the intestinal environment can be improved through the intake of the bifidobacterium.

### BRIEF DESCRIPTION OF DRAWINGS

Fig. 1 is a graph that compares the suppression of MuRF1 expression in the inventive and comparative strains.
Fig. 2 is a graph that compares the suppression of MuRF1 expression in the inventive strain and a type strain.

### DESCRIPTION OF EMBODIMENTS

The invention will now be described in detail.

### 1. Bifidobacterium breve strain N106 (NITE BP-03231)

The bifidobacterium in the present invention is *Bifidobacterium breve.* The symbol N106 in the present invention is a number independently assigned to the strain by Nissin Foods Holdings Co. Ltd. The strain was first isolated by the inventor from infant feces.

The *Bifidobacterium breve* strain N106 is deposited under the following conditions:
(1) Name of depositary: Patent Microorganisms Depositary Center of National Institute of Technology and Evaluation (NITE)
(2) Contact: Room 122, 2-5-8 Kazusa-Kamatari, Kisarazu, Chiba 292-0818, Japan
(3) Trustee number: NITE BP-03231
(4) Indication for identification: N106
(5) Consignment date: June 18, 2020

The bacteriological characteristics of *Bifidobacterium breve* strain N106 are shown in Tables 1 and 2 below. The bacteriological characteristics are determined based on the method described in Bergey's manual of systematic bacteriology Vol. 2 (1986). Table 1 shows the shape and other characteristics related to this strain, and Table 2 shows the results of physiological and biochemical characterizations using Api 20A (Biomérieux). In Table 2, the symbol "+" indicates positive and the symbol "-" indicates negative.

**[Table 1]**

| | | |
|---|---|---|
| Cultivation temperature (°C) | | 37°C |
| Shape of cells | | Bacillus |
| | | 0.7-0.8 × 2.0-4.0 µm |
| Gram stain | | Positive |
| Colony form | Culture medium | GAM agar medium |
| | Incubation time | 72 hours |
| | Diameter | 1.0-2.0 mm |
| | Color | Cream |
| | Shape | Circular |
| | Apophysis | Lenticular |
| | Fringe | Entire margin |
| | Surface | Smooth |
| | Transparency | Translucent |
| | Viscosity | Buttery |
| Spores | | - |
| Motility | | - |
| Catalase reaction | | - |
| Growth temperature test | 37°C | + |
| | 45°C | + |

| | | |
|---|---|---|
| +: Positive, -: Negative | | |

**[Table 2]**

| | Reaction/Enzyme | Result | | Reaction/Enzyme | Result |
|---|---|---|---|---|---|
| 1 | Production of indole | - | 11 | Hydrolysis of gelatine | - |
| 2 | Urease | - | 12 | Hydrolysis of esculin | - |
| 3 | Glucose fermentation | + | 13 | Glycerol fermentation | - |
| 4 | Mannitol fermentation | + | 14 | Cellobiose Fermentation | + |
| 5 | Lactose fermentation | + | 15 | Mannose fermentation | + |
| 6 | Saccharose fermentation | + | 16 | Meletitose Fermentation | + |
| 7 | Maltose fermentation | + | 17 | Raffinose fermentation | + |
| 8 | Salicin fermentation | + | 18 | Sorbitol fermentation | + |
| 9 | Xylose fermentation | + | 19 | Rhamnose fermentation | + |
| 10 | Arabinose fermentation | - | 20 | Trehalose fermentation | + |

### 2. Evaluation of suppression of MuRF1 expression

*Bifidobacterium breve* strain N106 of the present invention is highly active in suppressing the expression of the muscle atrophy gene, MuRF1, as shown in the experimental examples below. The suppression of MuRF1 gene expression was evaluated by the following testing method.

### <Preparation of bifidobacterial suspension>

Samples (bifidobacterial suspension) used in the evaluation of suppression of MuRF1 expression were cultured in a GAM medium (product name "GAM Broth": Nippon Suisan Kaisha, Ltd.) shown in Table 3 at 37°C for 24 hours. Anaeropak (Mitsubishi Gas Chemical Company, Inc.) was used for culture under anaerobic conditions. The proliferated bacteria were then collected by centrifugation. The collected bacteria were washed three times with sterile water, heat sterilized, and frozen. The bacteria were then freeze-dried using a freeze-dryer to yield dried bacteria powder. The dried powder was suspended in PBS as bifidobacterial suspension.

**[Table 3]**

| | |
|---|---|
| Peptone | 10 g |
| Soybean peptone | 3 g |
| Proteose peptone | 10 g |
| Digestive Serum powder | 13.5 g |
| Yeast extract | 5.0 g |
| Meat extract | 2.2 g |
| Liver extract | 1.2 g |
| Glucose | 3.0 g |
| Potassium dihydrogen phosphate | 2.5 g |
| Sodium chloride | 3.05 g |
| Soluble starch | 5.0 g |
| L-Cysteine hydrochloride | 0.3 g |
| Sodium thioglycolate | 0.3 g |
| Distilled water | 1000 mL |

### <Evaluation of suppression of MuRF1 expression>

The suppression of MuRF1 expression was evaluated in an in vitro study. Human Skeletal Muscle Myoblasts (Lonza K.K.) were used for the study. In accordance with the manufacturer's protocol, frozen cells were added to a growth medium (trade name "SkBM2": Lonza K.K.) for culture and growth. In each well of a 96 well plate was then seeded 2.0×10⁴ cells. The cells were cultured in a CO₂ incubator (5% CO₂, 37°C) for three days to allow them to adhere and grow on the plate, and then the growth medium was replaced with a differentiation medium (trade name "DMEM:F12 medium": prepared by Lonza K.K. with 2% horse serum) followed by cultivation for five days to differentiate into myotube cells. The above bifidobacterial suspension was added to the differentiation medium into a final concentration of 10 µg/mL. Dexamethasone, which is known to upregulate MuRF1 expression in skeletal muscle, was added to the medium so as to be 0.1 µM. After 24 hours of incubation, RNA was extracted using a Rneasy mini kit (QIAGEN). The extracted RNA was subjected to cDNA synthesis using ReverTra Ace qPCR RT Master Mix (Toyobo Co., Ltd.). Gene expression was measured by RT-PCR using THUNDERBIRD SYBR qPCR mix (Toyobo Co., Ltd.) and LightCycler 480 System II (Roche Diagnostics K.K.). RNA extraction, cDNA synthesis, and RT-PCR were performed in accordance with the protocols of the respective kits and instruments. The primer sequences used for the assay are listed in Table 4.

**[Table 4]**

| Primer Name | Sequence |
|---|---|
| MuRF1_F | TGGGGGAGCCACCTTCCTCT |
| MuRF1_R | ATGTTCTCAAAGCCCTGCTCTGTCT |
| RPLP0_F | GGAAACTCTGCATTCTCGCTTCCT |
| RPLP0_R | CCAGGACTCGTTTGTACCCGTTG |

### 3. Food and beverage

The bifidobacterium of the present invention can be used by being compounded in food and beverages. The bifidobacterium of the present invention is particularly suitable for use in beverages, for example, fermented milk and lactic acid bacteria beverages. According to the current Ministerial Ordinance on Standard of Ingredients for Milk and Dairy Products, the ingredient standards require a minimum of 1.0 × 10⁷ cfu/mL for fermented milk (with a solid content of nonfat milk of 8.0% or more) and dairy lactic acid bacteria beverages (with a solid content of nonfat milk of 3.0% or more) and a minimum of 1.0 × 10⁶ cfu/mL for lactic acid bacteria beverages (with a solid content of nonfat milk less than 3.0%), but the above bacteria counts can be achieved by growing in fermentation solutions such as milk or in the form of the final product. The bifidobacterium can also be used in dairy products such as butter, processed egg products such as mayonnaise, and pastries such as butter cake, in addition to fermented milk and lactic acid bacteria beverages containing the bifidobacterium. The bifidobacteria can also be suitably used in processed foods, such as instant noodles and cookies. In addition to these applications, the food products of the present invention may be in formulated forms (e.g., powder, granules, capsules, and tablets) of the bifidobacteria together with appropriate carriers and additives as necessary.

The bifidobacterium of the present invention is also useful to be compounded in foods for specified health uses and nutritional supplements in addition to general beverages and foods.

The bifidobacterium of the present invention can also be applied in cosmetics fields, such as lotion, pharmaceuticals fields, such as intestinal regulators, daily necessities fields, such as toothpaste, and animal feed and plant fertilizer fields, such as silage, animal feed, and plant liquid fertilizer, other than foods.

### INDUSTRIAL APPLICABILITY

The bifidobacterium (*Bifidobacterium breve* strain N106) has a high activity for suppressing the expression of a muscle atrophy gene, MuRF1.

### [Examples]

Examples of the present invention will now be described, but the present invention is not limited to the following examples.

### <Example 1> Evaluation of suppression of MuRF1 expression

The activity of the *Bifidobacterium breve* strain N106 of the present invention and 11 comparative strains owned by Nissin Foods Holdings Co. Ltd. were evaluated for suppression of MuRF1 expression.

The inventive strain, a type strain, and a comparative strain were each incubated at 37°C for 24 hours in a GAM medium (trade name "GAM Broth": Nippon Suisan Kaisha, Ltd.) as shown in Table 3. Anaeropac (Mitsubishi Gas Chemical Company, Inc.) was used to culture the bacteria under anaerobic conditions. The bacteria were then collected by centrifugation. The collected bacteria were washed three times with sterile water, heat sterilized, and frozen. The bacteria were then freeze-dried using a freeze-dryer to yield dried bacteria powder. The dried powder was suspended in PBS as bifidobacterial suspension.

Human Skeletal Muscle Myoblasts were then seeded in a 96-well plate at 2.0×10⁴ cells/well and were cultured for three days. After the medium was changed to a differentiation medium, the cells were cultured for five days to be differentiated into myotube cells. Each sample group was then added to the corresponding medium into a final concentration of 10 µg/mL. Dexamethasone was also added to the medium into 0.1 µM. After 24 hours of incubation, RNA was extracted from the cells and gene expression was measured by RT-PCR. A control was prepared that contained only dexamethasone but not contained the bifidobacterium suspension.

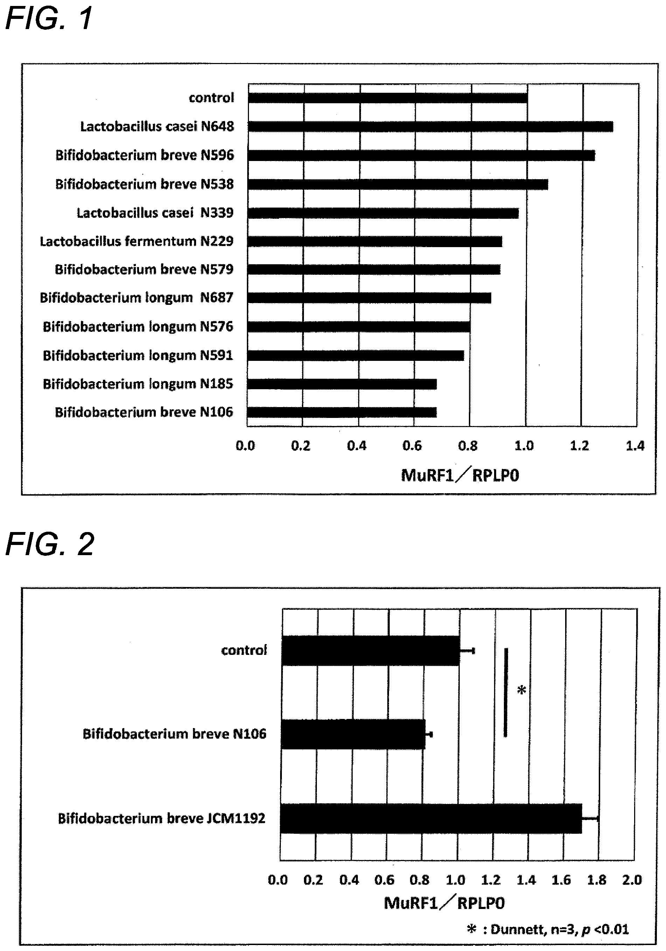

Fig. 1 shows the results of the addition of each sample group. The results indicate the MuRF1/RPLP0 ratio, with the control set to "1" for comparison.

Fig. 1 demonstrates that some bifidobacteria promote the expression of MuRF1, while others suppress the expression. Of these, strains N106 and N185 showed high activity in suppression of MuRF1 expression.

### <Example 2> Evaluation of the superiority of Bifidobacterium breve strain N106

The *Bifidobacterium breve* strain N106 was then evaluated in comparison with the type strain, *Bifidobacterium breve* JCM1192, to confirm the superiority of the *Bifidobacterium breve* strain N106. The test was performed three times as in Example 1, and the average value was used for comparison. The results are shown in Fig. 2.

Fig. 2 evidentially shows that the expression of MuRF1 was significantly suppressed in the group containing *Bifidobacterium breve* strain N106 compared to the control group.

As described above, *Bifidobacterium breve* strain N106 has a function that effectively suppresses the expression of the muscle atrophy gene MuRF1.

## Claims

1. A bifidobacterium belonging to *Bifidobacterium breve* species that suppresses expression of a muscle atrophy gene.

2. The bifidobacterium according to claim 1, wherein the muscle atrophy gene is a MuRF1 gene.

3. The bifidobacterium according to claim 1 or 2, wherein the bifidobacterium is *Bifidobacterium breve* strain N106 (NITE BP-03231).

4. A food or drink containing the bifidobacterium according to any one of claims 1 to 3.
